# EUROPEAN PATENT SPECIFICATION

(11) **EP 0 315 079 B1**
(45) Date of publication and mention of the grant of the patent: **01.03.1995**
(21) Application number: 88118025.1
(22) Date of filing: 28.10.1988
(51) Int. Cl.: A61K 9/10, A61K 9/50

(54) **Drug carriers**
Arzneimittelträger
Supports de médicaments

(30) Priority: 28.10.1987 JP 272770/87
(43) Date of publication of application: 10.05.1989
(73) Proprietor: NIPPON SHINYAKU COMPANY, LIMITED, Minami-ku Kyoto-shi Kyoto 601 (JP)
(72) Inventor: Sugiyama, Makoto, Yamashina-ku Kyoto 607 (JP); Okita, Atsuhiko, Otsu 520-02 (JP); Seki, Junzo, Ibaragi 567 (JP)
(74) Representative: Alber, Norbert

(56) References cited:
- EP-A- 0 167 825
- EP-A- 0 211 258
- EP-A- 0 274 961
- WO-A-87/01035
- DE-A- 3 225 706
- The Lipids, H.J.Deuel jr., Interscience Publishers, Inc.New York Interscience Publishers Ltd. London (1951), pp. 1 - 7

## Description

The present invention relates to an improved drug carrier in order to improve delivery of a drug contained therein from the blood stream or an applied site into lesional tissue.

Various investigations have been hitherto made on drug carriers for improving delivery of a drug contained from the blood stream or an applied site to a lesional tissue. For example, there is a method for utilizing liposome prepared by phospholipid ("Drug Carriers in Biology and Medicine" (1979), Ed. by G. Gregoriadis, Academic Press).

According to this method, however, the method encounter defects that (1) there are many problems in stability of liposome enveloping an aqueous phase with a lipid bilayer during its storage, (2) in the case of administration into blood, almost all liposomes are taken up into a tissue with a developed reticulo- entothelial system (RES) such as liver, spleen, etc. so that they are difficult to be distributed to other cells or tissues, etc. These are believed to be because liposome has a structure wherein the inner and outer aqueous phases are separated from each other by a phospholipid bilayer and liposome is thus unstable to various forces. An increase in particle diameter due to aggregation was also known as a defect during its storage.

According to investigations in recent years, there is known a technique in which various drugs are dissolved in a fat emulsion having a particle diameter of 0.2 »m composed of soybean oil and yolk lecithin heretofore used clinically as fluid supplementation for purposes of supplementing nutrients and the solution is used; good results obtained thereby for the object described above (SAISHIN IGAKU (Latest Medicine), 40, 1806-1813 (1980)). This carrier is characterized in that it has no aqueous phase inside and can be extremely stably stored as compared to liposomes.

However, the carrier has a property that is readily taken up into the aforesaid reticulo- endothelial system such as liver, etc. Such a rapid metabolism was desired as a high calorie fluid supplementation but involved problems of causing poor distribution of a drug into other tissues as a drug carrier suited for the object described above and was not necessarily desirable.

Further, an art of using fat emulsion wherein 90% of it is 100 ± 30 nm as a carrier for pharmaceuticals is disclosed in Japanese Laid Open 63/500456. However this art is also characterized in accumulation on reticulo- endothelial system such as liver and spleen and, as already described, there is a problem in the delivery of drug to other tissues.

As a means for solving the foregoing problems, a technique of applying serum lipoproteins composed of a simple lipid (including sterols, same as in the present specification), a complex lipid and an apolipoprotein as a drug carrier was known (Japanese Patent Application Laid-Open No. 60-163824). However, this carrier is to introduce a drug into cells by physiological and specific recognition of the lipoprotein. Therefore, the carrier is rapidly transferred into tissue via its receptor so that disappearance from the blood is relatively rapid. For this reason, transfer into a tissue having a poor receptor activity is not always sufficient. Furthermore, apolipoprotein is indispensable as its constituent so that the technique involves a defect in industrial technique that results in high production costs.

Further, an attempt of making fat emulsion of 200 nm particle size more fine is known (cf. Japanese Laid Open 62/29511). However, in this art, yolk lecithin used is a little and, accordingly, the resulting microparticles are recoagulated with an elapse of time whereby there is a problem instability. Moreover, there is a disadvantage as to the stability in vivo, so said art is not desirable in terms of delivery to other tissues.

From document D1 (EP-A-0 211 258) microemulsion composistions for parenteral administration are known wherein the microemulsion consists essentially of a discontinuous droplet phase comprising a pharmaceutically acceptable lipid and/or lipophilic drug which contain from about 0.6% to about 10% pharmaceutically acceptable emulsifier by weight as the sole emulsifier in the composition.

Document D2 (WO-A-87/01035) describes pharmaceutical microemulsions, which can be used for parenteral administration of fat-soluble pharmaceuticals and vitamins.

Document D3 (DE-A-32 25 706) describes liquid microemulsions which contain co-emulgators (sinthetic surface-active agents) as essential components.

From document D4 (EP-A-0 167 825) lipid nanopellets with an average diameter range of 80 to 800 nm are known which should be used as a carrier system for drugs for parenteral use comprising a lipid mixture such as triglycerides, a surface-active agent such as a phosphor lipid and an active agent. No specific ratio with respect to the components and the surface layer can be derived.

In general, a drug administered moves and distributed in the body due to properties inherently possessed by its drug molecule. Then, the drug reaches the site of action to exhibit its pharmacological effects. In this case, it is desired that the drug is concentrated only on the site necessary for exhibiting the pharmacological effects but the drug is generally distributed over the entire body and the drug moves also to a site that does not require the drug. This sometimes becomes a cause for side effects. Thus, it becomes important and necessary to improve disposition of a drug in the body.

In view of the circumstances described above, the present inventors have continued to investigate novel drug carriers, (1) without affecting pharmacological activities per se of a drug, (2) capable of effective delivery of a drug into focal tissue, (3) capable of reducing uptake by the reticulo- endothelial system, (4) capable of durating a concentration of a drug in blood and (5) capable of reducing a dose of drug required. As a result, they have finally succeeded in accomplishing the present invention.

The feature of the present invention lies in the following points.
(1) The drug carrier is a fat emulsion constituted by two of a lipophilic substance as the core and a lipophilic substance having covered the surface thereof and is not in such a form as in liposome wherein the aqueous phase is present inside;
(2) In the drug carrier, a drug is present in a state of dispersion, dissolution, formation of mixed micelles or chemical binding with lipid; and,
(3) A particle diameter is in a range of not less than 5 nm to 100 nm.

Hereafter, these points will be described in detail.

The drug carrier of the present invention has a form as a stable fat emulsion. It is desired that its particle diameter be in a range of not less than 5 nm to 100 nm, in order to avoid uptake into the reticulo- endothelial system. By super finely dividing the drug carrier, its blood concentration can be maintained in a higher level than in a fat emulsion having a diameter of about 0.2 »m. Particularly preferred is a diameter of 100 nm or less. This is because the drug carrier can easily exudate out of blood vessel through a region in which vasopermeability is accentuated.

It is known that various regions called pore systems (it is said that a small pore system having a diameter up to 9 nm and a large pore system having a diameter of 25 to 70 nm are present and it is known that the vasopermeability is further increased in various focal regions including neoblastic vessels) or other slits between cells are present in blood vessels and vasopermeability is accentuated in various focal regions including inflammation, tumor and atherosclerosis. In such a region, the drug carrier of the present invention is selectively exudated out of blood vessel in large amounts and transferred into focal tissue. At the same time, the drug contained in the drug carrier is also delivered into the lesion. By this, the drug can easily be delivered to the focal region selectively so that a drug concentration at the focal region increases and its effect can be enhanced. Further by applying thee drug carrier of the present invention, a drug can be administered simultaneously with lipid so that sustained releasability of a drug and lymphotropic properties of a drug can be improved. The drug carrier of the present invention also subjects to phagocytable properties to phagocyte.

The characteristic feature of the present invention lies in using super finely divided lipid as a drug carrier. By the super finely divided particles, the problems described above that not only the foregoing effects are exhibited but also uptake by the reticulo- endothelial system is prohibited, can be solved at once. By this, an effect of keeping a drug concentration in blood can also be obtained.

The drug carrier in accordance with the present invention is characterized in that by using larger amounts of the surface layer (compound lipid) in its proportion to the core (simple lipid), as compared to the conventional high calorie fluid supplementation, comprising soybean oil and yolk lecithin, super finely divided particles are realized.

In order to facilitate super finely divided particles in the drug carrier of the present invention, it is desired that the content of the surface layer (complex lipid) be in a range of 15% to 70%. This is because a surface area of the core in the drug carrier is increased by super finely division so that it is necessary to increase an amount of the compound lipid in order to cover the core as a surface layer and stabilize the emulsion. In the case of using less than 15% of compound lipid, it is unavoidable to intermingle particles having a diameter of 0.2 »m or more; in the case of using more than 70% of compound lipid, it is unavoidable to intermingle liposome particles. By this compositional constitution, a stable emulsion of super finely divided lipid emulsion can be obtained and is usable as an extremely excellent drug carrier, which has been made clear by the present invention for the first time.

That is, it is considered that the drug carrier of the present invention would be in the form of a fat emulsion composed of a substance as the core and a substance as the surface layer, wherein (1) the substance constituting the core of the fat emulsion is a simple lipid and a ratio of said substance in the drug carrier is 30 to 85%; (2) the substance constituting the surface layer of the fat emulsion is a compound lipid and a ratio of said substance in the drug carrier is 15 to 70%; and by possessing properties (1) and (2) at the same time, the drug carrier containing a drug therein having a mean particle diameter of between 5-100 nm can be obtained.

In the present invention, it is required that the mode of containing the drug should be dispersing or dissolving in the drug carrier, forming a mixed micelle with a constituent(s) of the drug carrier or chemically binding with a constituent(s) of the drug carrier so that the applied drug is not readily released from the drug carrier.

As the lipid used in the drug carrier of the present invention, mention may be made of a simple lipid, derived lipid or a compound lipid derived from natural animal, vegetable or mineral or a mixture thereof. Examples include a simple lipid, derived lipid or a compound lipid derived from yolk, soybean, cotton, linseed, corn, sesame, peanut, safflower, bovine tissue, hog tissue, sheep tissue or a simple lipid, derived lipid or a compound lipid purely synthetically prepared.

Examples of the simple lipid include neutral lipids such as refined soybean oil, cotton seed oil, linseed oil, sesame oil, corn oil, peanut oil, safflower oil, triolein, trilinolain, tripalmitin tristearin trimyristin triarachidonin. The simple lipid also embraces cholesterol derivatives such as cholesteryl oleate, cholesteryl linoleate, cholesteryl myristate, cholesteryl palmitate, cholesteryl arachidonate. This is because neutral lipids are relatively easily decomposed by various lipases present in blood vessel endothelium, whereas cholesterol derivatives are decomposed only with difficulty by these enzymes and

As the derived lipid, mention may be, for example, cholesterol, fatty acids such as stearic palmitic acid, oleic acid, linoleic acid, linolenic acid, eicosapentaenoic acid and derivatives thereof, squalene. They may also be used as emulsification aids. Furthermore, oily compounds such as azone, may be exemplified.

As the compound lipid, mention may be made of, for example, phospholipids derived from yolk, soybean, bovine tissue, hog tissue. Examples of phospholipids include phosphatidyl choline, phosphatidyl ethanolamine, phosphatidyl serine, phosphatidyl inositol, which are exemplified by yolk phosphatidyl choline, soybean phosphatidyl choline, dipalmitoyl phosphatidyl choline, dimyristoyl phosphatidyl choline, distearoyl phosphatidyl choline, dioleoyl phosphatidyl choline, dipalmitoyl phosphatidyl inositol. Products obtained by hydrogenation of these phospholipids may also be used. Among them, a representative preferred example is yolk phosphatidyl choline. As the glycolipid, mention may be made of cerebroside, etc. Steryl glucosides, e.g., β-sitosteryl-β-D-glucoside can also be exemplified. Furthermore, lipids having a charge such as stearyl amine, dicetyl phosphate, phosphatidic acid may also be used to impart a surface charge to the drug carrier.

The drug to which the present invention is applicable may be any drug so long as it is pharmaceutically acceptable but is not particularly limited. Even a drug that is insoluble or sparingly soluble can also be used. In the present invention, the drug readily forms a complex with the carrier.

In a water-soluble drug, the drug carrier of the present invention can be formed by using the drug chemically bound to the constituents (for example, lipid) of the carrier.

Even though a drug is unstable in the body and is thus incapble of administration so far, such a drug can also be readily administered by using the drug carrier of the present invention. The drug contained in the drug carrier of the present invention is present in oil droplets of lipids in such a state that is shielded from the surrounding environment so that enzymatic or non-enzymatic decomposition can be prevented.

The drug to which the drug carrier of the present invention is applicable is not particularly limited, as described above. Examples include an antiinflammatory agent, an analgesic, an anti-allergic agent, an antibiotic, a chemotherapeutic agent, an anti-cancer agent, antiviral agent, anti-artherosclerosis, an anti-lipemic agent, an antiulcer agent, an immunoregulator, a vaccine, a radical scavenger, a bronchodilator, a hypnotic, a tranquilizer, a topical anesthesia, a diagnostic. Specific examples of these drugs are anticancer agents such as Ancitabine, fluorouracil, mitomycin C, mitomycin C farnesylamide, mitomycin C farnesylacetic amide, Carmofur, Futraful palmitate, 5-fluorouracil myristate, Adriamycin, Daunomycin, Aclarubicin, Maclarubicin, Vinblastine, Vincristine, Cytarabine fatty acid esters, Mitotane, Estramustine; antiviral agents such as Dichloroflavan; steroidal agents (for example, Dexamethasone palmitate, hydrocortisone palmitate, Prednisolone palmitate, Dexamethasone stearate, Methylprednisolone, Paramethasone, Fluocinolone acetonide, Vectamethasone propionate, Hydrocortisone fatty acid esters, Aldosterone, Spironolactone) and non-steroidal agents (for example, Ibuprofen, Flufenamic acid, Ketoprofen, Phenacetin, Antipyrine, Aminopyrine, Phenylbutazone indoleacetate, Biphenylylpropionic acid derivatives, Indometacin, Indometacin ethoxycarbonylmethyl ester, Indometacin stearyl ester, Sodium aurothiomalate cetyl ester, Diclofenac, acetylsalicylic acid and derivatives thereof). Antiallergic agents such as Tranylast, Ketotifen, Azelastine, may also be used. As antibiotics and chemotherapeutic agents, mention may be made of, for example, tetracyclines, Erythromycin, Midecamycin, Amphotericin, Nalidixic acid, Griseofulvin, Minocyclin. As examples of prostaglandines, there may be used PGE1, PGA1, PGA1 alkyl esters, PGE1 alkyl esters, PGE1 derivatives, PGI2 derivatives, PGD2 derivatives. Antihistaminic agents such as Diphenhydramine, Orphenadirine, Chlorphenoxamine, Chlorpheniramine, Promethazine, Mecridine, Cyproheptadine, Loxatidine acetate are mentioned. Furthermore, as topical anesthesia, mention may be made of Lidocaine, Benzocaine, Dantrolene, Cocaine, Tetracaine, Piperocaine, Mepyracaine, and derivatives thereof. There are also mentioned hepatic disorder improving agents (for example, Marotirate, Glycyrretinic acid, ethyl acetyl-glycyrretinate, methyl glycyrretinate), antiulcer agents (for example, Farnesol, Geraniol, Gefarnate, Teprenone, Plaunotol, Sofarcon). There are also agents acting on central nerves (for example, Phenobarbital, Methaqualon, Heroin, Diazepam, Medazepam, Frazepam, Clotiazepam, Etizolam, Mecridine, Bucridine, Adiphenine, Methamphetamine, Imipramine, Chlorimipramine, Amitriptyline, Mianserin, Trimethadione, Phensuximide, Tetrabenzamide, Benzquinamide, Camphor, Dimorphoramine, Strychnine, Chlorpromazine, Promethazine, Prochlorperazine, Mequitazine, Triflupromazine, Levomepromazine, Difenidol and derivatives thereof). Also cerebrovasodilators (for example, Cinnarizine) may be mentioned. As bronchodilators, mention may be made of Vestphyllin and other theophylline derivatives, methylephedrine. Anticholinergic agents (for example, Benztropine, Physostigmine, Atropine, Scopolamine), parasympathetic blockers (for example, Oxyphencyclimine, Pirenzemine, Etomidrine), calcium blockers (for example, Diltiazem, Nifedipine, Verapamil), α-blockers (for example, Dibenzamine, Phenoxybenzamine), antitussive agents (for example, Noscapine, Dextromethorphan, Pentoxyverine, Benproperine), agents for treating prostatic hypertrophy (for example, Gastron, Oxendelone), agents for treating glaucoma (for example, Pilocarpine), agents acting on smooth muscle (for example, Sparteine, Papaverine) and agents for treating hyperlipemia (for example, Chlorfibrate, Cimfibrate, Probucol). In addition, mention may be made of, for example, amino acids, vitamins, Dilazep, Ubidecarenone, Flavoxate, Cyclosporin, vaccines for influenza, etc., Dibenzthione, Diphenylpyraline, Phenovalinium, Metadione, Tofisopam, Limonen).

Antioxidants (for example, tocopherol, flavone derivatives, gallic acid derivatives, coffee acid derivatives, Goshipol, Sezamol, oxyfatty acid, camphene, Cineol, Rosmanol, Eugenols, Filozurucine catechins, lignan homologues, p-coumaric acid, sterols, terpenes, bromophenol, etc.) can also form the drug carrier of the present invention as one of the constituents.

Further, guaiazulene, essential oil type crude drugs (for example, apricot kernel oil, fennel oil, thyme oil, terepentine oil, eucalyptus oil, palm oil, poppy seed oil, tsubaki oil, peppermint oil, clove oil, mint oil, sage oil and other componentns for spicy crude drugs) can also form the drug carrier of the present invention as one of the constituent factor of the drug carrier of the present invention.

As diagnostics, mention may be made of, for example, a compound labeled with a radioisotope, a radioactive drug or iodated poppy oil fatty acid esters as X ray contrast materials.

The drug to which the drug carrier of the present invention is applicable is not particularly limited as described above but when viewed from characteristics possessed inherently as the drug carrier, drugs which take part in inflammation, tumor, blood vessel or immune or lymphoid system are generally desired.

The drug concentration in the drug carrier of the present invention can be suitably varied within a range that the content does not exceed 85% in the drug carrier, according to biological activity of the drug. Further the concentration of the drug carrier of the present invention in medical preparations obtained using the drug carrier of the present invention can be suitably varied and is optional, if desired.

Upon preparation of the drug carrier of the present invention and medical preparations using the same, various methods for preparing emulsion hitherto performed can apply. For example, they can be prepared according to a method which comprises sufficiently finely dividing all constituents including a drug by means of a homogenizer of Manton-Gaurin type, a microfluidizer, a ultrasonic wave homogenizer. They can be also prepared according to a method which comprises solubilizing the constituents using a surface active agent (for example, bile acid), a water-soluble solvent (for example, ethanol, polyethylene glycol) and then removing the surface activity agent or water-soluble solvent by dialysis or gel filtration.
Fatty acids or derivatives thereof may also be added as emulsification aids. Furthermore, the drug carrier and its medical preparations may also be obtained by adding a drug to a fat emulsion free from particles having a diameter of 200 nm or more previously prepared by the aforesaid methods.

The shape and particle diameter of the drug carrier of the present invention can easily be confirmed by an electron microscope, a particle diameter analyzer of light scattering type, filtration through a membrane filter. As optional components for medical preparations using the drug carrier of the present invention, mention may be made of additives and auxiliary substances used for ordinary injections. Examples are an antioxidant, a preservataive, a stabilizer, an isotonic agent, a buffer. Required and optimum amounts of these additives and auxiliary substance can be varied depending upon their purposes.

The drug carrier of the present invention obtained as described above can be sterilized (for example, sterilized by filtration or with steam under high pressure in an autoclave), if necessary, and sealed in an ampoule together with nitrogen gas. Also if necessary, the drug carrier may be freeze dried. The freeze dried drug carrier of the present invention can be restored by adding an appropriate solution thereto.

The drug carrier of the present invention is generally administered intravenously to humans and animals but if necessary, can also be administered intraarterially, intramuscularly and subcutaneously.

Furthermore, the drug carrier of the present invention can also be used as an eye drop, a nose drop, an oral agent and a suppository. In this case, additives such as pharmaceutically acceptable bases and excipients can be exemplified as optional components.

### (Effects)

According to the present invention, a value for drug availability can be markedly enhanced. The effects of the drug carrier of the present invention can be summarized in that the prior art problems are overcome, (1) delivery of a drug into the focal lesion is improved, (2) uptake by the reticulo- endothelial system is prevented, (3) a blood concentration of drug contained therein can be maintained, (4) stability during storage is ensured, (5) production costs are reduced. These effects have been achieved by the present invention for the first time.

It is also a characteristic that the major constituents of the drug carrier of the present invention are therapeutically acceptable lipids conventionally used for therapy in the clinical field so that they can be used extremely safely.

### (Examples)

Hereafter the present invention will be explained in more detail, by referring to examples relating to preparation of the drug carrier of the present invention.

### Example 1

To 27 mg of triolein were added 38 mg of yolk lecithin and 10 mg of guaiazulene (antiinflammatory agent) and, 10 ml of physiological saline was added to the mixture. Using a probe type ultrasonic wave homogenizer (Branson Sonifier Model 185), the mixture was subjected to a ultrasonic wave treatment for 60 minutes under ice cooling. The formed drug carrier containing guaiazulene was blue and clear. A mean particle diameter of the drug carrier was 26.4 nm when measured by a light scattering particle diameter measurement device. Further in observation by an electron microscope, the drug carrier was recognized to be uniform, spherical ultra finely divided particles. Any lipid bilayer membrane as in liposome was not noted. It was also noted that the drug carrier passed by 100% through a filtering membrane of 0.2 »m and did not contain particles of 0.2 »m or more.

### Example 2

Yolk lecithin, 2.5 mg and 10 mg of guaiazulene were added and, 10 ml of physiological saline was added to the mixture. Using a probe type ultrasonic wave homogenizer (Branson Sonifier Model 185), the mixture was subjected to a ultrasonic wave treatment for 60 minutes under ice cooling. The formed drug carrier containing guaiazulene had a mean particle diameter of 48.4 nm according to an apparatus for measuring optical scattering particles. It was also noted that the drug carrier passed by 100% through a filtering membrane of 0.2 »m and did not contain particles of 0.2 »m or more.

### Example 3

To 100 mg of triolein were added 100 mg of yolk lecithin and 4 mg of a compound (Dexamethasone palmitate) obtained by chemically binding a fatty acid with Dexamethasone (antiinflammatory agent) and, 10 ml of 0.24 M glycerin aqueous soltuion was added to the mixture. Using a probe type ultrasonic wave homogenizer (Branson Sonifier Model 185), the mixture was subjected to a ultrasonic wave treatment for 60 minutes under ice cooling. The formed drug carrier containing Dexamethasone palmitate was slightly bluish white and clear. A mean particle diameter of the drug carrier was 29.9 nm when measured by a light scattering particle diameter measurement device.

It was also noted that the drug carrier passed by 100% through a filtering membrane of 0.2 »m and did not contain particles of 0.2 »m or more.

### Example 4

To 80 mg of triolein were added 20 mg of cholesteryl linoleate, 100 mg of yolk lecithin and 4 mg of Dexamethasone palmitate. Then, 10 ml of 0.24 M glycerin aqueous soltuion was added to the mixture. Using a probe type ultrasonic wave homogenizer (Branson Sonifier Model 185), the mixture was subjected to a ultrasonic wave treatment for 60 minutes under ice cooling. The formed drug carrier containing Dexamethasone palmitate was slightly bluish white and clear. A mean particle diameter of the drug carrier was 30.6 nm when measured by a light scattering particle diameter measurement device. It was also noted that the drug carrier passed by 100% through a filtering membrane of 0.2 »m and did not contain particles of 0.2 »m or more.

### Example 5

To 100 mg of cholesteryl linolate were added 100 mg of yolk lecithin and 4 mg of Dexamethasone palmitate. Then, 10 ml of 0.24 M glycerin aqueous soltuion was added to the mixture. Using a probe type ultrasonic wave homogenizer (Branson Sonifier Model 185), the mixture was subjected to a ultrasonic wave treatment for 60 minutes under ice cooling. The formed drug carrier containing Dexamethasone palmitate was slightly bluish white and clear. A mean particle diameter of the drug carrier was 22.7 nm when measured by a light scattering particle diameter measurement device. It was also noted that the drug carrier passed by 100% through a filtering membrane of 0.2 »m and did not contain particles of 0.2 »m or more.

### Example 6

To 100 mg of triolein were added 100 mg of yolk lecithin and 10 mg of Diphenhydramine (antihistaminic agent). Then, 10 ml of 0.24 M glycerin aqueous soltuion was added to the mixture. Using a probe type ultrasonic wave homogenizer (Branson Sonifier Model 185), the mixture was subjected to a ultrasonic wave treatment for 60 minutes under ice cooling. The formed drug carrier containing Diphenhydramine was slightly bluish white and clear. A mean particle diameter of the drug carrier was 31.6 nm when measured by a light scattering particle diameter measurement device. It was also noted that the drug carrier passed by 100% through a filtering membrane of 0.2 »m and did not contain particles of 0.2 »m or more.

### Example 7

To 100 mg of triolein was added 100 mg of yolk lecithin. Then, 10 ml of 0.24 M glycerin aqueous soltuion was added to the mixture. Using a probe type ultrasonic wave homogenizer (Branson Sonifier Model 185), the mixture was subjected to a ultrasonic wave treatment for 60 minutes under ice cooling. The formed drug carrier was slightly bluish white and clear. A mean particle diameter of the drug carrier was 47.2 nm when measured by a light scattering particle diameter measurement device. It was also noted that the drug carrier passed by 100% through a filtering membrane of 0.2 »m and did not contain particles of 0.2 »m or more.

A compound (Vinblastine palmitate), 500 »g, obtained by chemically binding a fatty acid with Vinblastine (anticancer agent) was added to the drug carrier obtained above. The mixture was gently mixed and stirred for 6 hours to take the drug up into the drug carrier. Thus, the drug carrier containing the drug was obtained.

A compound (5-Fluorouracil palmitate), 500 »g, obtained by chemically binding a fatty acid with 5-Fluorouracil (anticancer agent) was added to the drug carrier obtained above. The mixture was gently mixed and stirred for 6 hours to take the drug up into the drug carrier. Thus, the drug carrier containing the drug was obtained.

A compound (Cytarabine levulinate), 500 »g, obtained by chemically binding a fatty acid with Cytarabine (anticancer agent) was added to the drug carrier obtained above. The mixture was gently mixed and stirred for 6 hours to take the drug up into the drug carrier. Thus, the drug carrier containing the drug was obtained.

### Example 8

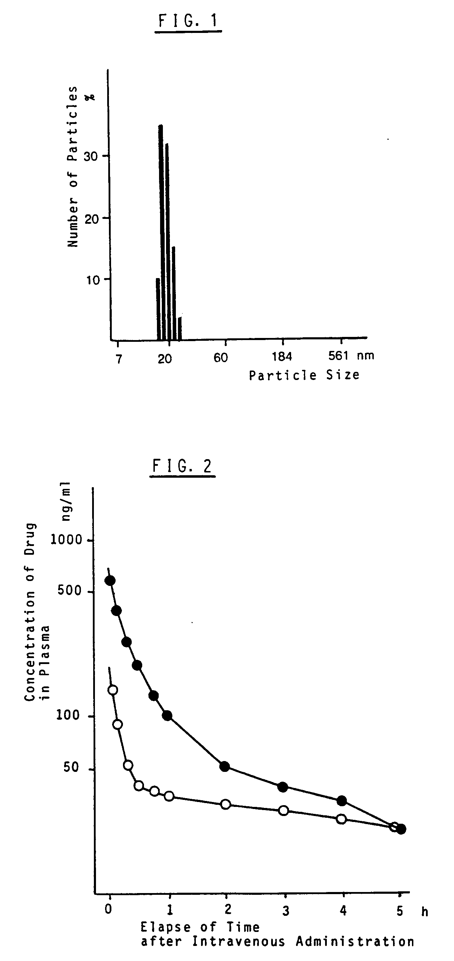

To 80 mg of triolein were added 20 mg of cholesteryl linoleate and 100 mg of yolk lecithin. Then, 10 ml of 0.24 M glycerin aqueous soltuion was added to the mixture. Using a probe type ultrasonic wave homogenizer (Branson Sonifier Model 185), the mixture was subjected to a ultrasonic wave treatment for 60 minutes under ice cooling. The formed drug carrier was slightly bluish white and clear. A mean particle diameter of the drug carrier was 19.1 nm when measured by a light scattering particle diameter measurement device. The analytical results are shown in Fig. 1. It was also noted that the drug carrier passed by 100% through a filtering membrane of 0.2 »m and did not contain particles of 0.2 »m or more.

### Example 9

To 20 mg of refined soybean oil was added 20 mg of yolk lecithin. Then, 10 ml of 0.24 M glycerin aqueous soltuion was added to the mixture. Using a probe type ultrasonic wave homogenizer (Branson Sonifier Model 185), the mixture was subjected to a ultrasonic wave treatment for 60 minutes under ice cooling. The formed drug carrier was slightly bluish white and clear. A mean particle diameter of the drug carrier was 16.1 nm when measured by a light scattering particles diameter measurement device. It was also noted that the drug carrier passed by 100% through a filtering membrane of 0.2 »m and did not contain particles of 0.2 »m or more.

Furthermore, a drug carrier was prepared in a manner similar to above, except for using 40 mg of refined soybean oil. The formed drug carrier was slightly bluish white and clear. A mean particle diameter of the drug carrier was 37.7 nm when measured by a light scattering particle diameter measurement device. It was also noted that the drug carrier passed by 100% through a filtering membrane of 0.2 »m and did not contain particles of 0.2 »m or more.

### Example 10

To 10 g of soybean oil was added 10 g of yolk lecithin. Then, 1 liter of 0.24 M glycerine aqueous soltuion was added to the mixture. Using a microfluidizer, the mixture was emulsified. It was noted that the formed drug carrier passed by 100% through a filtering membrane of 0.2 »m and did not contain particles of 0.2 »m or more.

### [Test on stability of the drug carrier of the present invention]

### Test Example 1-1

The sample obtained in Example 1 was sealed in a brown ampoule of 1 ml volume together with nitrogen gas. A forced deterioration test was performed at 60°C for 4 weeks in a conventional manner. The residual rate of guiazulene was 98.3% more and it was confirmed that the drug carrier of the present invention had effects in stability of the drug.

### Test Example 1-2

The samples obtained in Examples 1, 3 and 4 described above were sealed, respectively in a brown ampoule of 1 ml volume together with nitrogen gas. After a sterilizing treatment of the ampoules with steam under high pressure in an autoclave, a particle diameter of each sample was measured by a light scattering particle diameter measurement device. There was no significant difference between prior to and after the treatment. Neither aggregation nor increase in the particle diameter was noted. Further they were stored at 4°C for 6 months but no change such as aggregation was noted.

### Test Example 1-3

The sample obtained in Example 3 described above was freeze dried in a conventional manner. Thereafter, distilled water for injection was added to the sample followed by stirring to restore. Then a particle diameter of the sample was measured by a light scattering particle diameter measurement device. A mean particle diameter was 28.3 nm. There was neither significant aggregation nor increase in the particle diameter was noted but the sample was uniformly dispersed.

### [Test on utility of the present invention]

### Test Example 2-1

The drug carrier of the present invention containing ³H-labeled Dexamethasone palmitate prepared in a manner similar to Example 3 was used as a test sample. As a comparative sample, a fat emulsion having a diameter of 0.2 »m as the prior art was used. This comparative sampled was obtained by adding 10 ml of 0.24 M glycerin aqueous soltuion to 4 mg of ³H-labeled Dexamethasone palmitate, 100 mg of refined soybean oil and 12 mg of yolk lecithin.

The test sample and the comparative sample were intravenously administered to rats. Then, a change in blood concentration was examined.

Change in the total radioactivity in plasma when the test sample and the comparative sample were intravenously administered to the tail vein of SD strain male rats (weighing about 210 g) in a dose of 0.05 mg/kg calculated as Dexamethasone is shown in Fig. 2, by calculating into Dexamethasone. The comparative sample rapidly disappeared from plasma but disappearance of the test sample was gentle. Half life periods in the distribution phases were 10.5 minutes and 5.5 minutes, respectively.

### Test Example 2-2

Delivery of the drug into an inflammatory region induced by carrageenin edema was compared between the test sample and the comparative sample, using the drug carrier of the present invention ³H-labeled Dexamethasone palmitate prepared in a manner similar to Example 4 as a test sample and the same comparative sample as used in Test Example 2-1.

**Table 1**

| Delivery of Drug into Carrageenin Inflammatory Region | | | |
|---|---|---|---|
| | | Test Sample | Comparative Sample |
| Paw with inflammation | (ng) | 475 ± 175 | 154 ± 17 |
| | (ng/g) | 204 ± 53 | 64 ± 8 |
| Control paw | (ng) | 164 ± 19 | 89 ± 24 |
| | (ng/g) | 94 ± 8 | 52 ± 14 |
| Edema region Plasma | (ng/g) | 538 ± 142 | 94 ± 42 |
| | (ng/g) | 448 ± 38 | 122 ± 12 |
| Indication is (mean ± standard deviation) | | | |

Carrageenin edema was induced by subcutaneously administering 0.1 ml of 0.5% λ-carrageenin to SD strain male rats (weighing about 195 g) at one paw heel. Two hours after the administration of carrageenin, the test sample and the comparative sample were intravenously administered in the tail vein in a dose of 0.5 mg/kg when calculated as Dexamethasone. Sixty minutes after the intravenous administration, blood was collected from the aorta in the abdomen to obtain plasma. At the same time, the paw with inflammation and the opposite paw (control paw) were cut off from the ankle joint. Each radioactivity was measured after treating with a sample oxidizer.

In Table 1, with respect to the test sample, large amounts of the drug were transferred into the inflammatory region (edema region) and strong accumulation onto the inflammatory region was noted, as compared to the comparative sample. A drug concentration of 5.7 times that of the comparative sample was noted in the edema region induced by inflammation.

### Test Example 2-3

Table 2 indicates the results of comparison in delivery of a drug into the hydrothorax and the major organs in rats with pleurisy model, using the same test sample and comparative sample as used in Test Example 2-2 described above.

2% λ-Carrageenin, 0.1 ml, was administered to SD strain male rats (weighing about 300 g) at the thoracic cavity. Two and half hours after the administration of carrageenin, the test sample and the comparative sample were intravenously administered in the tail vein in a dose of 1.25 mg/kg when calculated as Dexamethasone. Thirty minutes after the intravenous administration, blood was collected from the aorta in the abdomen and fluid in the thoracic cavity was washed out with physiological saline to make 10 ml. Its radioactivity was determined. At the same time, the major organs were ectomized. Each radioactivity was measured after treating with a sample oxidizer.

**Table 2**

| Transfer into Inflammatory Region and Major Tissues | | |
|---|---|---|
| | Test Sample | Comparative Sample |
| Fluid in the thoracic cavity (»g) | 2.65 | 0.68 |
| Diaphragm (»g/g) | 1.06 | 0.68 |
| Spleen (»g/g) | 3.05 | 27.34 |
| Liver (»g/g) | 7.71 | 17.84 |
| Heart (»g/g) | 1.53 | 1.53 |
| Lung (»g/g) | 2.36 | 1.93 |
| Kidney (»g/g) | 2.66 | 1.37 |
| Plasma (»g/ml) | 10.07 | 2.37 |
| Indication is a mean value when converted into Dexamethasone. | | |

In Table 2, with respect to the test sample, large amounts of the drug were delivered into the inflammatory region (hydrothoracic region) and strong accumulation onto the inflammatory region was noted, as compared to the comparative sample. A drug concentration of 3.9 times that of the comparative sample was noted in the fluid in the thoracic cavity. In distribution into the major organs, the test sample showed extremely low transfer in transfer into organs having developed reticulo- endothelial system such as liver and spleen.

### Test Example 2-4

The same test sample and comparative sample as used in Test Example 2-2 described above were intravenously administered to BALB/C male mice (weighing about 25 g). Thirty minutes after the administration, a concentration of the unchanged drug and a concentration of Dexamethasone as its metabolite were determined in plasma and liver. The dose was made 5 mg/kg when calculated as Dexamethasone.

Table 3 shows each concentration of the unchanged drug (Dexamethasone palmitate, its concentration was converted into Dexamethasone) and its metabolite (Dexamethasone) separately determined quantitatively.

In the case of the test sample, a concentration in plasma was high and distribution in the liver was low. Further the test sample was mostly present in plasma as the unchanged drug. In the case of using the drug carrier of the present invention, maintenance of blood concentration of the drug and a preventing effect in uptake into the reticulo- endothelial system are obviously noted.

**Table 3**

| | Test Sample (»g/ml, g) | Comparative Sample (»g/ml, g) |
|---|---|---|
| Unchanged drug in plasma | 36.3 ± 2.2 | 7.7 ± 1.6 |
| Dexamethasone in plasma | 4.6 ± 0.5 | 5.2 ± 0.9 |
| Unchanged drug in Liver | not detectable | not detectable |
| Dexamethasone in Liver | 24.0 ± 1.2 | 41.0 ± 1.3 |
| Liver/Plasma ratio in concentration (total amount) | 0.6 ± 0.0 | 3.0 ± 0.5 |
| Indication is (mean ± standard deviation) | | |

### Test Example 2-5

With respect to the same test sample and comparative sample as used in Test Example 2-2 and a physiological saline solution of Dexamethasone phosphate, their pharmacological effects were examined using carrageenin edema inhibition as the index.

λ-Carrageenin (0.5%, 1 ml) was subcutaneously administered to SD strain male rats (weighing about 160 g) at one paw heel. Thirty minutes after, the test sample, the comparative sample and Dexamethasone phosphate were intravenously administered in the tail vein. For the control group, physiological saline was administered. A volume of the paw was measured prior to the administration of carrageenin and 5 hours after the administration in a conventional manner to determine an edema inhibition ratio.

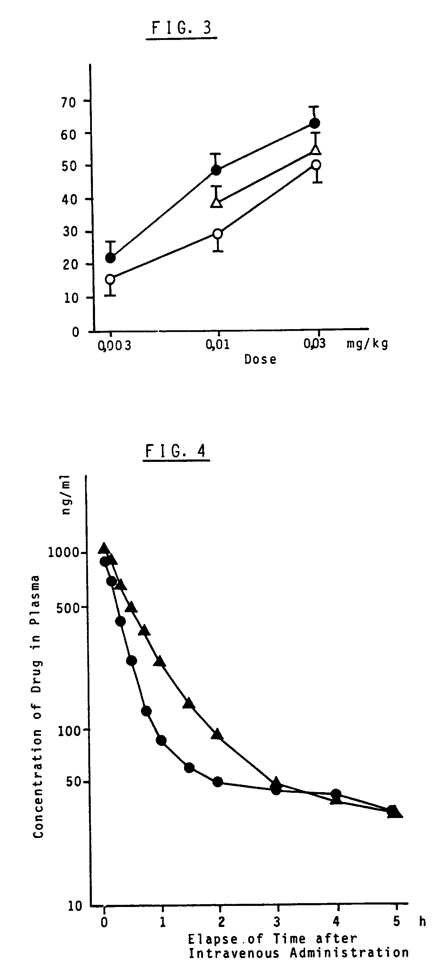

Fig. 3 shows its dose-response curve (indicated as Dexamethasone calculated). Fig. 4 shows a 50% edema inhibition dose (ED₅₀).

It is apparent that the test sample had an antiinflammatory activity by about twice that of the other two samples, even in inflammation of this kind which was not improved with the comparative sample as the prior art. That is, the effect of the drug carrier of the present invention was confirmed as an effect of enhancing the drug effect. It is thus clear that this is because the drug is efficiently delivered to the focal lesion by using the drug carrier of the present invention.

**Table 4**

| 50% Edema Inhibition Dose | |
|---|---|
| | ED ₅₀ (mg/kg) |
| Test Sample | 0.012 |
| Comparative Sample | 0.031 |
| Dexamethasone phosphate | 0.023 |
| (Indication is made by conversion into Dexamethasone) | |

### Test Example 2-6

With respect to the same test sample and comparative sample as used in Test Example 2-2 and a physiological saline solution of Dexamethasone phosphate, their pharmacological effects were examined by the inhibition of carrageenin granuloma as the index. Further, weights of thymus and adrenals were examined.

λ-Carrageenin (2.0%, 4.0 ml) was subcutaneously administered to SD strain male rats (weighing about 160 g) at the back. From on day 5, each sample was intravenously administered to the tail vein once daily for 3 days 3 times in total. A dose of the drug administered was made 0.05 mg/kg/once. For the control group, physiological saline was administered. Eight days after, granuloma, thymus and adrenal were ectomized and their weights were measured.

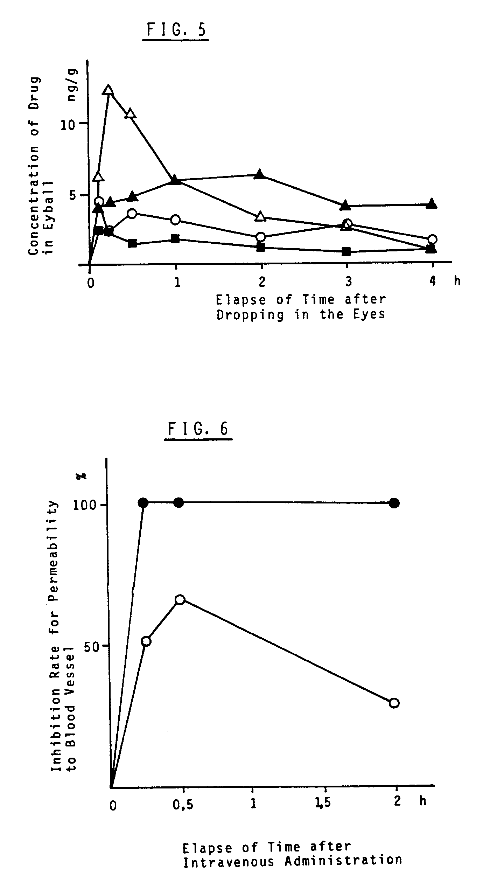

It is noted from Fig. 5 that the test sample showed obviously strong granuloma formation inhibition activity as compared to the comparative sample and Dexamethasone phosphate and also showed less atrophy in the thymus and the adhrenals. That is, it is shown that the test sample had a strong pharmacological effect but less side effects.

**Table 5**

| Weights of granuloma, thymus and adrenal | | | |
|---|---|---|---|
| | granuloma | Thymus | Adrenal |
| Control | 20.5 ± 5.4 g | 416.0 ± 63.3 mg | 55.2 ± 9.6 mg |
| Test Sample | 10.9 ± 1.4 g | 205.0 ± 57.2 mg | 44.5 ± 7.2 mg |
| Comparative Sample | 15.5 ± 2.6 g | 149.8 ± 31.3 mg | 39.6 ± 2.7 mg |
| Indication is (mean ± standard deviation) | | | |

### Test Example 2-7

In order to confirm deliverability to the tumor region, a test was performed.

P388 leukemia cells, 10⁶ cells, were subcutaneously transplanted to CDF1 male mice (weighing about 25 g) at the right front limb. Six days after, the right front limb was cut out and provided for the experiment 5 days after. By this treatment, metastatic cancer model into the right upper arm and the right axilla lymph nodes was obtained. As the test sample, the drug carrier of the present invention prepared in Example 8 using ³H-labeled cholesteryl linoleate was used. As the comparative sample, a fat emulsion having a diameter of 0.2 »m , composed of refined soybean oil and yolk lecithin hitherto known in which ³H-labeled cholesteryl linoleate had been incorporated was used. The test sample and the comparative sample were intravenously administered into the tail vein and 60 minutes after, the right upper arm and the right axilla lymph nodes in which tumor matastasis was noted were ectomized. Further as a non-metastatic lymph node, the left upper arm and the left axilla lymph nodes were simultaneously ectomized. Each radioactivity was measured.

As shown in Table 6, the drug carrier of the present invention was transferred to the tumor region in a concentration as high as twice or more. In the comparative sample, such a selective delivery in a high concentration wad not noted.

**Table 6**

| Delivery to Metastatic Lymph Node Tumor | | |
|---|---|---|
| | Test Sample | Comparative Sample |
| Metastatic lymph nodes | 2.60 ± 0.87 | 0.91 ± 0.27 |
| Non-metastatic lymph nodes | 1.04 ± 0.27 | 0.86 ± 0.39 |
| Indication is (% of dose/g, mean ± standard deviation) | | |

### Test Example 2-7a.

A test confirming a tranfer to tumor region was conducted.

S-180 tumor cells (1 x 10⁶) were inoculated subcutaneously to the abdominal skin of ddY strain mice (body weight: ca. 25 g). After 6 days, diameter of the tumor became about 1 cm and it was subjected to a test.

As to a sample, the pharmaceutical carrier of the present invention in Example 8 prepared from ³H-labelled cholesteryl linoleate was used. As a control, ³H-labelled cholesteryl linoleate was made incorporated into fat emulsion comprising yolk lecithin and soybean oil of 0.2 micron diameter and the product was used.

Both sample and cntrol were administered into tail vein, then tumor was taken out after 15 minutes, 1 hour and 24 hours, and the radioactivity was determined.

As shonw in Table 6a, the carrier of the present invention was transferred, in each time tested, to the tumor region at the concnetration of about three times as much as compared with the control.

**Table 6a**

| Transfer to Solid Tumor | | |
|---|---|---|
| Time [hr] | Tested Sample | Control |
| 0.25 | 1.37 ± 0.34 | 0.35 ± 0.14 |
| 1 | 1.64 ± 0.21 | 0.54 ± 0.13 |
| 24 | 6.59 ± 0.38 | 2.96 ± 1.22 |
| The figures are in % of dose/g; average ± standard deviation. | | |

### Test Example 2-8

For purposes of confirming stability of the drug carrier of the present invention in the body in which cholesteryl linoleate was the core, the drug carrier of the present invention obtained in Example 5 was used as a test sample and as a comparative sample, the drug carrier of the present invention obtained in Example 4 was used. These samples were intravenously administered to rats, respectively. Change in blood concentration was determined. Samples prepared using ³H-labeled Dexamethasone palmitate were used as the respective samples.

Change in total radioactivity in plasma when the test sample and the comparative sample were intravenously administered to SD male rats (weighing about 250 g) in tail vein in a dose of 0.05 mg/kg when calculated as Dexamethasone is shown in Fig. 4, by converting into Dexamethasone. The test sample disappeared from plasma more gently than the comparative sample. Half lives for the disappearance in the distribution phase were 21.6 minutes and 11.5 minutes, respectively.

### Test Example 2-9

The test samples obtained in Examples 3, 4 and 5 and the comparative sample used in Test Example 2-1 were mixed with rat plasma, respectively to examine the stability. A concentration of the sample in plasma was made 23 »g/ml when calculated as Dexamethasone. As shown in Table 7, the amount of the unchanged drug (Dexamethasone palmitate) remained after incubation at 37°C for 90 minutes, namely, stability in plasma, was obviously superior in the drug carrier of the present invention to the comparative sample.

In addition, it was also confirmed that the use of cholesteryl linoleate the core of the drug carrier of the present invention increased the stability depending upon its content.

**Table 7**

| Stability in Plasma | |
|---|---|
| | Remaining amount of Unchanged Drug |
| Test Sample obtained in Example 3 | 39.8% |
| Test Sample obtained in Example 4 | 47.5% |
| Test Sample obtained in Example 5 | 68.1% |
| Comparative Sample of Test Example 2-1 | 20.1% |

### Test Example 2-10

After applying an eye drop of test preparation to the eye of ddY mice (weighing about 30 g) under anesthesia with pentobarbital, a drug concentration in the eyeball was measured and deliverability of the drug into the eyeball was examined.

Test preparations are four below.
- Test sample - (1): drug carrier of the present invention containing antiinflammatory guaiazulene obtained in Example 1
- Test sample - (2): drug carrier of the present invention containing antiinflammatory guaiazulene obtained in Example 2
- Comparative sample - (1): fat emulsion having a diameter of 0.2 »m composed of soybean oil and yolk lecithin as the prior art in which guaiazulene had been incorporated
- Comparative sample - (2): fat emulsion having a diameter of 0.2 »m composed of soybean oil and yolk lecithin as the prior art in which sodium guaiazulene-3-sulfonate as a water soluble derivative of guaiazulene had been mixed and dissolved.

A dose was made 5 »g/eye when calculated as guaiazulene. After applying to the eye, the eyeball was ectomized in a definite time. After immediately washing with physiological saline, the eyeball was homogenized and the drug was determined by high performance liquid chromatography.

Change in drug concentration in the eyeball is shown in Fig. 5. The test samples all showed better deliverability to the eyeball than the comparative samples. It is evident that delivery of the drug into the eyeball was improved in the case of using the drug carriers of the present invention.

### Test Example 2-11

Using the drug carrier containing guaiazulene obtained in Example 1 as a test sample and water soluble derivative of guaiazulene, sodium guaiazulene-3-sulfonate as a comparative sample, these samples were applied to the eye of Japanese white rabbits (weighing about 3 kg) to examine delivery of the drug to the aqueous humor. Thirty minutes after the eyedropping, the aqueous humor was collected and a drug concentration was measured. The results are shown in Table 8. Only in the case of using the drug carrier of the present invention, delivery of the drug to the aqueous humor was noted.

**Table 8**

| Delivery of Drug to Aqueous Humor after Application to the Eye | |
|---|---|
| Test Sample | 3.47 ± 3.31 |
| Comparative Sample | not detectable |
| Indication is (mean ± standard deviation) | |

### Test Example 2-12

Using the drug carrier of the present invention containing antihistaminic Diphenhydramine obtained in Example 6 as a test sample and a Diphenhydramine hydrochloride solution in physiological saline as a comparative sample, a preventive action against accentenuation of vasopermeability induced by intracutaneous administration of histamine was examined.
The test sample or the comparative sample were intravenously administered to SD strain male rats (weighing about 300 g). After a definite time period, 10 mg of Evans Blue was intravenously administered and at the same time, histamine hydrochloride (1 »g/50 »l) was intracutaneously injected to the abdominal skin. Further 30 minutes after, The skin was peeled apart to quantitatively determine Evans Blue exudated into the skin. After the skin was solubilized with 3 ml of conc. hydrochloric acid, 3 ml of 10% benzarconium chloride was added to the solution, Evans Blue was extracted with 5 ml of chloroform. An amount of Evans Blue exudated into the skin was determined by absorbance at 620 nm in the chloroform layer.

Fig. 6 shows time-dependent change of the inhibition of the vasopermeability induced by intracutaneously injecting histamine 15, 30 and 120 minutes after administration of both samples, doses of which were made 2 mg/kg when calculated as Diphenhydramine. The test sample showed the maximum effect already 15 minutes after the administration. The effect was continued up to 2 hours. On the other hand, in the comparative sample, its inhibition rate was lower than the test sample. The comparative sample showed the maximum effect 30 minutes after the administration and the effect was then decreased. The test sample showed theiinhibition of the vasopermeability by 3 times or more than the comparative sample 2 hours after the administration. By the results, it is shown that the test sample not only enhances the drug effects but also has an effect of duration in the drug action.

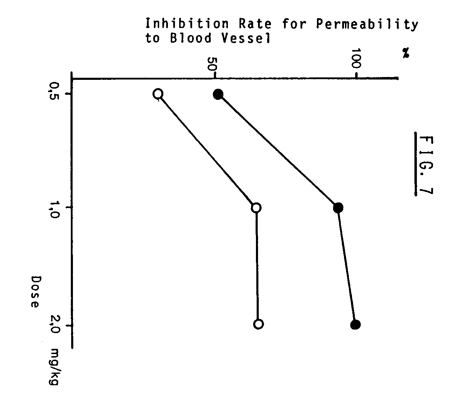

Fig. 7 shows a dose-response curve showing the inhibition of vasopermeability obtained 30 minutes after administration of the samples. It is evident that the test sample are excellent in the inhibition of the vasopermeability as compared to the comparative sample.

### 4. Brief Description of the Drawings

Fig. 1 shows results of a particle diameter of the drug carrier of the present invention prepared in Example 8 measured with a light scattering particle diameter measurement device, wherein the vertical axis represents the number of particles and the abscissa represents a particle diameter with a logarithmic scale.

Fig. 2 shows change of the total radioactivity in plasma when the test sample and the comparative sample examined in Test Example 2-1 were intravenously administered to rats, wherein the vertical axis represents a concentration of the drug calculated as Dexamethasone (ng/ml) and the abscissa represents a time passage (hour) after administration: a curve connected with ● and a curve connected with o represent the test sample and the comparative sample, respectively.

Fig. 3 is a dose-response curve of antiinflammatory activity obtained using a carrageenin edema inhibition rate as the index, when the test sample and the comparative sample examined in Test Example 2-2 were intravenously administered to rats, wherein the vertical axis represents a inhibitory rate of carrageenin edema by % and the abscissa represents a dose of the drug calculated as Dexamethasone with a logarithmic scale: a curve connected with ●, a curve connected with △ and a curve connected with o represent the test sample, Dexamethasone phosphate and the comparative sample, respectively.

Fig. 4 represents change of the total radioactivity in plasma when the test sample and the comparative sample examined in Test Example 2-8 were intravenously administered to rats, wherein the vertical axis represents a concentration (ng/ml) of Dexamethasone calculated from the radioactivity and the abscissa represents passage of time (hour) after administration: a curve connected with ▲ and a curve connected with ● represent the test sample and the comparative sample, respectively.

Fig. 5 represents amount of the drug delivered to the eyeball after applying the two test samples and the two comparative samples examined in Test Example 2-10, wherein the vertical axis represents a concentration (ng/ml, calculated as guaiazulene) of the drug in the eyeball and the abscissa represents passage of time (hour) after application to the eye: a curve connected with ▲, a curve connected with △, a curve connected with ■ and a curve connected with o represent Test Sample - (1), Test Sample - (2), Comparative Sample - (1) and Comparative Sample - (2), respectively.

Fig. 6 represents the time courses of the inhibition of the vasopermeability when the test sample and the comparative sample examined in Test Example 2-12 were intravenously administered to rats, wherein the vertical axis represents the inhibition of the vasopermeability by per cent and the abscissa represents passage of time (hour) after administration of sample.

A curve connected with ● and a curve connected with o represent the test sample and the comparative sample, respectively.

Fig. 7 represents a dose-response curve in the inhibition of the vasopermeability when the test sample and the comparative sample examined in Test Example 2-12 were intravenously administered to rats, wherein the vertical axis represents the inhibition of the vasopermeabillty by per cent and the abscissa represents dose of the drug calculated as Diphenhydramine hydrochloride with a logarithmic scale.

A curve connected with ● and a curve connected with o represent the test sample and the comparative sample, respectively.

## Claims

1. A parenteral drug carrier in the form of a fatty emulsion for avoiding uptake into a tissue with a developed reticuloendothelia system such as liver, which contains a drug and has a mean particle diameter of 5 to 100 nm, and which comprises a core and a surface layer, characterized in that:
(1)
the substance constituting the core is a simple lipid, and a ratio of the substance in the drug carrier is 30 to 85 %,
(2)
the substance constituting the surface layer is a compound lipid, and a ratio of the substance in the drug carrier is 15 to 70 %,
and having properties (1) and (2) simultaneously.

2. A parenteral drug carrier as claimed in claim 1, wherein the substance constituting the core is the simple lipid selected from the group consisting of a neutral lipid, or a sterol ester, or a mixture thereof.

3. A parenteral drug carrier as claimed in claim 1, wherein the compound lipid is a phospholipid(s).

4. A parenteral drug carrier as claimed in claim 1, wherein the derived lipid selected from the group consisting of a fatty acid(s) and cholesterol, may be added as emulsification aids.

5. A parenteral drug carrier as claimed in claim 1, wherein the drug is an anti-inflammatory and analgesic agent.

6. A parenteral drug carrier as claimed in claim 1, wherein the drug is an anti-cancer agent.

7. A parenteral drug carrier as claimed in claim 1, wherein the drug is an antibiotic and chemotherapeutic agent.

## Patentansprüche

1. Parenteraler Arzneimittelträger in Form einer Fettemulsion zur Verhinderung der Aufnahme in ein Gewebe mit einem entwickelten reticuloendothelialen System wie der Leber, der ein Arzneimittel enthält und einen durchschnittlichen Teilchendurchmesser von 5 bis 100 nm aufweist und der einen Kern und eine Oberflächenschicht umfaßt,
**dadurch gekennzeichnet,** daß
(1) die den Kern bildende Substanz ein einfaches Lipid und ein Verhältnis der Substanz in dem Arzneimittelträger 30 bis 85% sind,
(2) die die Oberflächenschicht bildende Substanz ein zusammengesetztes Lipid und ein Verhältnis der Substanz in dem Arzneimittelträger 15 bis 70% sind, und
er die Eigenschaften (1) und (2) gleichzeitig aufweist.

2. Parenteraler Arzneimittelträger nach Anspruch 1,
**dadurch gekennzeichnet,** daß
die den Kern bildende Substanz das aus einem neutralen Lipid oder einem Sterinester oder einer Mischung davon ausgewählte einfache Lipid ist.

3. Parenteraler Arzneimittelträger nach Anspruch 1,
**dadurch gekennzeichnet,** daß
das zusammengesetzte Lipid ein Phospholipid(e) ist(sind).

4. Parenteraler Arzneimittelträger nach Anspruch 1,
**dadurch gekennzeichnet,** daß
ein sich aus Fettsäure(n) und Cholesterin ausgewähltes hergeleitetes Lipid als Emulgierhilfsmittel zugesetzt sein kann.

5. Parenteraler Arzneimittelträger nach Anspruch 1,
**dadurch gekennzeichnet,** daß
das Arzneimittel ein entzündungshemmendes und analgesisches Mittel ist.

6. Parenteraler Arzneimittelträger nach Anspruch 1,
**dadurch gekennzeichnet,** daß
das Arzneimittel ein Anti-Krebsmittel ist.

7. Parenteraler Arzneimittelträger nach Anspruch 1,
**dadurch gekennzeichnet,** daß
das Arzneimittel ein antibiotisches und chemotherapeutisches Mittel ist.

## Revendications

1. Support de médicament parentéral sous forme d'une émulsion grasse pour éviter l'absorption dans un tissu présentant un système de réticuloendothelie développé tel que le foie, qui contient un médicament et présente un diamètre de particule moyen de 5 à 100 nm, et qui comporte un noyau et une couche de surface, caractérisé en ce que
(1)
la substance constituant le noyau est un lipide simple, et un ratio de la substance dans le support de médicament est de 30 à 85 %,
(2)
la substance constituant la couche de surface est un composé lipidique, et un ratio de la substance dans le support de médicament est de 15 à 70 %,
et présentant les propriétés (1) et (2) simultanément.

2. Support de médicament parentéral selon la revendication 1, dans lequel la substance constituant le noyau est un simple lipide sélectionné parmi le groupe consistant en un lipide neutre, ou un ester de stérol ou un mélange des deux.

3. Support de médicament parentéral selon la revendication 1, dans lequel le composé lipidique est un phospholipide.

4. Support de médicament parentéral selon la revendication 1, dans lequel le lipide dérivé sélectionné parmi le groupe consistant en un acide gras et du cholestérol peut être ajouté en tant qu'adjuvant d'émulsification.

5. Support de médicament parentéral selon la revendication 1, dans lequel le médicament est un agent anti-inflammatoire et analgésique.

6. Support de médicament parentéral selon la revendication 1, dans lequel le médicament est un agent anticancéreux.

7. Support de médicament parentéral selon la revendication 1, dans lequel le médicament est un agent antibiotique ou chimiothérapeutique.
